(19) Europäisches Patentamt
European Patent Office
Office européen des brevets

(11) Veröffentlichungsnummer: **0 064 605**
**B1**

# (12) EUROPÄISCHE PATENTSCHRIFT

(45) Veröffentlichungstag der Patentschrift: **10.10.84**

(21) Anmeldenummer: **82103008.7**

(22) Anmeldetag: **08.04.82**

(51) Int. Cl.³: **C 07 C 69/88,** C 07 C 69/92, C 07 C 67/30, C 07 C 43/205, C 07 C 41/00, C 07 C 121/75, C 07 C 120/00, A 61 K 7/46, C 11 B 9/00

## (54) Verfahren zur Herstellung von Resorcinderivaten sowie Verwendung bestimmter Resorcinderivate als Riechstoffe.

(30) Priorität: **29.04.81 DE 3116913**

(43) Veröffentlichungstag der Anmeldung: **17.11.82 Patentblatt 82/46**

(45) Bekanntmachung des Hinweises auf die Patenterteilung: **10.10.84 Patentblatt 84/41**

(84) Benannte Vertragsstaaten: **CH DE FR GB LI NL**

(56) Entgegenhaltungen:
**DE - A - 2 653 177**
**FR - A - 2 161 202**
**US - A - 4 173 982**

**JOURNAL OF ORGANIC CHEMISTRY, Band 39, Nr. 25, 1974, Columbus, M.S. KABLAOUI "The aromatization of cyclic ketones. II. Novel synthesis of substituted dihydroxybenzenes", Seiten 3696 bis 3698**

(73) Patentinhaber: **BASF Aktiengesellschaft, Carl-Bosch-Strasse 38, D-6700 Ludwigshafen (DE)**

(72) Erfinder: **Thoemel, Frank, Dr., Leberstrasse 17, D-6940 Weinheim (DE)**
Erfinder: **Hoffmann, Werner, Dr., Ringstrasse 11C, D-6701 Neuhofen (DE)**

**EP 0 064 605 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein Verfahren zur Herstellung von Resorcinderivaten der allgemeinen Formel I

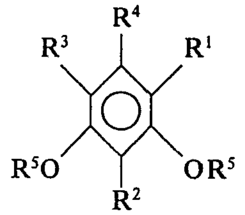

(I)

in welcher $R^1$ eine Alkoxycarbonylgruppe mit einem niederen Alkylrest, eine Nitrilgruppe, einen Alkylrest oder Wasserstoff bedeutet, und $R^2$ bis $R^5$ für Wasserstoff oder einen niederen Alkylrest stehen sowie die Verwendung von Resorcinderivaten der allgemeinen Formel III

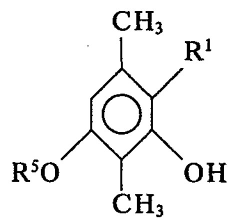

(III)

in welcher $R^1$ eine Alkoxycarbonylgruppe mit einem niederen Alkylrest oder eine Nitrilgruppe und $R^5$ einen niederen Alkylrest bedeuten, als Riechstoffe in Riechstoffkompositionen.

Eichenmoosextrakte spielen in der Parfümerie eine wichtige Rolle, hauptsächlich bei der Herstellung von Chypre- oder Fougèrekompositionen. Zu den wichtigsten Geruchsträgern des natürlichen Eichenmooses gehören $\beta$-Resorcylsäurederivate. Es sind daher immer wieder Anstrengungen unternommen worden, solche Verbindungen zu erhalten. Dabei kann es sich sowohl um die natürlichen Inhaltsstoffe des Eichenmooses (vergl. R. Ter Heide et al, Perfumes, Cosmetiques, Aromes 1975 [3], 61) als auch um rein synthetische Produkte handeln.

Als Ausgangsprodukte für synthetische $\beta$-Resorcylsäurederivate bieten sich die synthetisch leicht zugänglichen Dihydro-$\beta$-resorcylsäurederivate der allgemeinen Formel IV an, in welcher $R^6$ einen niederen Alkylrest und $R^2$

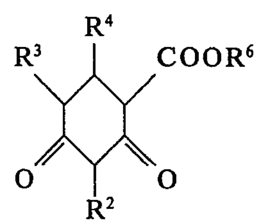

(IV)

bis $R^4$ Wasserstoff oder einen niederen Alkylrest darstellen. Diese Verbindungen lassen sich entweder durch Kondensation von $\alpha,\beta$-ungesättigten Carbonsäureestern mit $\beta$-Ketoestern (vgl. A. Sonn, Ber. Chem. Ges. 62 [2] [1929] 3012) oder durch Kondensation von Malonsäureestern mit $\alpha,\beta$-ungesättigten Ketonen (vgl. U. Steiner und B. Wilhelm, Helv. Chim. Acta 35, [1952] 1752) herstellen.

Das Hauptproblem bei der Synthese der angeführten Eichenmoosriechstoffe vom Resorcin-Typ ist die Aromatisierung der Verbindungen IV. A. Sonn (loc. cit.) benutzte zu diesem Zweck einen teuren Palladiumkatalysator. Gemäß der DE-AS 1 941 041 und der US-PS 3 634 491 werden Chlor oder chlorerzeugende Stoffe als Oxidationsmittel vorgeschlagen. Arbeitet man mit Brom in Eisessig (US-PS 3 884 843) so erhält man bromsubstituierte Aromaten, die noch enthalogeniert werden müssen. In der US-A-4 173 983, Beispiel 2, ist die Herstellung von 2,4-Dihydroxy-3,6-di-methyl-benzonitril durch Oxidierung der entsprechenden Dioxocyclohexanverbindung mit Chlor in essigsaurer Lösung beschrieben. Das Arbeiten mit Halogenen erfordert jedoch spezielle Apparaturen und bringt Probleme der Arbeits- und Umweltsicherheit mit sich. Verhältnismäßig einfach gelingt die Aromatisierung mit Hilfe von Acetanhydrid/Schwefelsäure (vgl. DE-OS 2 653 177). Es entstehen dabei jedoch zunächst die Diacetate, die in einer zusätzlichen Reaktionsstufe verseift werden müssen.

Es war daher die Aufgabe der Erfindung ein Verfahren zu entwickeln, nachdem die Resorcinderivate der Formel I unter Vermeidung der Nachteile der bekannten Verfahren auf einfache Weise und mit guten

Ausbeuten hergestellt werden können.

Gegenstand der Erfindung ist ein Verfahren zur Herstellung von Resorcinderivaten der allgemeinen Formel I

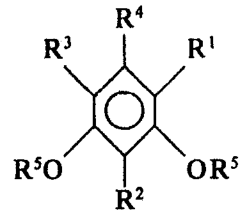

(I)

in welcher $R^1$ eine Alkoxycarbonylgruppe mit einem niederen Alkylrest, eine Nitrilgruppe, einen niederen Alkylrest oder Wasserstoff bedeutet und $R^2$ bis $R^5$ für Wasserstoff oder einen niederen Alkylrest stehen, das dadurch gekennzeichnet ist, daß man die entsprechenden Cyclohexyn-1,3-dione der allgemeinen Formel II

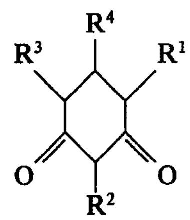

(II)

in welcher die Substituenten die oben angegebene Bedeutung haben, in Gegenwart katalytischer Mengen einer Kupferverbindung sowie in Gegenwart von 1 bis 10 mol, vorzugsweise 5 bis 10 mol, eines Halogenwasserstoffgases oder von 0,5 bis 5 mol, vorzugsweise 1 bis 3 mol Thionylchlorid pro mol II in einem praktisch wasserfreien Alkanol mit 1 bis 6 C-Atomen, praktisch wasserfreiem Tetrahydrofuran oder praktisch wasserfreiem Methyl-tert.-butylether als Lösungsmittel bei Reaktionstemperaturen von 0 bis 150°C, vorzugsweise 20 bis 100°C, mit Sauerstoff oder Sauerstoff enthaltenden Gasen umsetzt.

Besonders vorteilhaft gestaltet sich das Verfahren, wenn man die Umsetzung in Gegenwart katalytischer Mengen von Kupfer(I)oxid durchführt und/oder wenn man die Umsetzung in Gegenwart von Chlorwasserstoffgas, Bromwasserstoffgas oder von Thionylchlorid durchführt.

Gegenstand der Erfindung ist ferner die Verwendung von Resorcinderivaten der allgemeinen Formel III

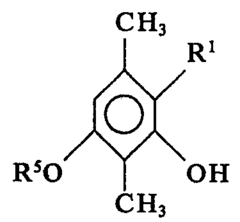

(III)

in welcher $R^1$ eine Alkoxycarbonylgruppe mit einem niederen Alkylrest, vorzugsweise eine Methoxycarbonylgruppe oder eine Nitrilgruppe und $R^5$ einen niederen Alkylrest bedeuten, als Riechstoffe.

Es war sehr überraschend, daß die Resorcinderivate der Formel I nach dem erfindungsgemäßen Verfahren mit besonders guten Ausbeuten hergestellt werden können.

Es war zwar aus der US-PS 3 859 365 bekannt, daß man alkylsubstituierte Phenole durch Umsetzen der entsprechenden 2-Cyclohexen-1-one mit Sauerstoff in Gegenwart von Kupfer(II)-chlorid, Salzsäure und einem mit Wasser mischbaren Lösungsmittel bei 20 bis 200°C in relativ guten Ausbeuten herstellen kann, jedoch erhält man bei Versuchen, die in der genannten US-PS beschriebene Verfahrensweise für die Umsetzung von Cyclohexenonen auf die Umsetzung von Cyclohexan-1,3-dionen zu übertragen, die Resorcinderivate der Formel I nur in außerordentlich geringen Ausbeuten. Beispielsweise erhält man bei der Umsetzung des 3,6-Dimethyl-β-dihydroresorcylsäuremethylesters gemäß den Verfahren dieser US-PS den 3,6-Dimethyl-β-resorcinsäure-methylester als einzigem Aromatisierungsprodukt in Ausbeuten von bestenfalls 17 % der Theorie. Überraschenderweise wurde gefunden, daß diese Umsetzung mit sehr guten Ausbeuten an dem Aromatisierungsprodukt verläuft, wenn man sie praktisch ohne Zusatz von Wasser zum Reaktionsgemisch durchführt.

Als Ausgangsverbindungen verwendet man vorzugsweise solche Cyclohexan-1,3-dione der Formel II, in denen $R^1$ eine Alkoxycarbonylgruppe mit 1 bis 4 C-Atomen, insbesondere eine Methoxycarbonylgruppe oder eine Nitrilgruppe bedeutet und $R^2$ bis $R^4$ für Wasserstoff oder eine Alkylgruppe mit 1 bis 4 C-Atomen, insbesondere Wasserstoff oder eine Methylgruppe stehen.

Als Katalysatoren sind organische oder anorganische Kupfer(I)-salze, Kupfer(II)-salze sowie Kupfer(I)oxid und Kupfer(II)oxid geeignet. Die Kupfersalze können noch Kristallwasser enthalten. Genannt seien beispielsweise CuCl, $CuCl_2 \times 2\ H_2O$, $CuBr_2$, $Cu(CH_3CO)_2$, $Cu_2O$, CuO und $Cu(NO_3)_2$.

Mit besonderem Vorteil verwendet man $Cu_2O$ als Katalysator. Der Katalysator wird im allgemeinen in Mengen von 0,01 bis 0,5 mol, vorzugsweise 0,05 bis 0,3 mol pro mol II eingesetzt.

Als Halogenwasserstoffgase verwendet man erfindungsgemäß mit Vorteil Chlorwasserstoffgas oder Bromwasserstoffgas oder aber Thionylchlorid. Die Halogenwasserstoffgase bzw. Thionylchlorid verwendet man im allgemeinen in mindestens molaren Mengen, bezogen auf das Cyclohexandion II, vorteilhaft in Mengen von 5 bis 10 mol für die Halogenwasserstoffgase und 1 bis 3 mol pro mol II für Thionylchlorid.

Als Lösungsmittel verwendet man bei den erfindungsgemäßen Verfahren niedermolekulare Alkanole wie Methanol, Ethanol, Propanol, Butanol, Amylalkohol, Isopropylalkohol, und tert. Butylalkohol oder aber Methyl-tert.-butylether. Für die Herstellung von Resorcinderivaten mit unveretherten Hydroxyl-Gruppen hat sich aber auch Tetrahydrofuran als geeignet erwiesen. Man muß mindestens soviel Lösungsmittel einsetzen, wie zur Auflösung der zu aromatisierenden Verbindung nötig ist. Dimethylformamid, aromatische oder aliphatische Kohlenwasserstoffe sowie Halogenkohlenwasserstoffe eignen sich nur sehr schlecht oder gar nicht.

Die Lösungsmittel verwendet man erfindungsgemäß in praktisch wasserfreier Form, d.h. man setzt Lösungsmittel ein, die keine nennenswerten Mengen an Wasser enthalten und sorgt dafür, daß dem Reaktionsgemisch keine nennenswerten Mengen an Wasser zugefügt werden. Wie Vergleichsversuche zeigen, bringen schon Zusätze von etwa 5% Wasser zum Lösungsmittel ganz beträchtliche Ausbeuteverluste mit sich. Die Wassermengen, die eventuell in Form von Kristallwasser mit einem Kupfersalzkatalysator eingebracht werden sowie das bei der Umsetzung gebildete Wasser beeinträchtigten die Umsetzung nicht wesentlich.

Die erfindungsgemäße Reaktion wird im allgemeinen bei Normaldruck und bei einer Temperatur unterhalb der Siedetemperatur bis zu der Siedetemperatur der eingesetzten Lösungsmittel durchgeführt. Gewöhnlich arbeitet man zwischen 20 und 100°C. Die Umsetzung ist prinzipiell aber auch in einem Druckgefäß unter Aufpressen von Luft oder Sauerstoff durchführbar.

Die zur Oxidation für ein mol Substrat erforderliche Luft- bzw. Sauerstoffmenge liegt zwischen 10 und 2000 Liter/Stunde, bevorzugt aber zwischen 40 und 1000 Liter/Stunde. In der Regel leitet man die Luft bzw. den Sauerstoff in das Reaktionsgemisch ein. Man kann aber auch so verfahren, daß man die Lösung der Ausgangsverbindung in eine Lösung des Katalysatorsystems eintropft und dabei gleichzeitig Luft oder Sauerstoff in das Reaktionsgemisch einleitet.

Die Reaktionsdauer kann entsprechend den gewählten Reaktionsbedingungen zwischen wenigen Stunden und einigen Tagen betragen. Mit Vorteil wählt man die Reaktionsbedingungen so, daß der Aromatisierungsprozeß nach spätestens 15 Stunden beendet ist.

In den meisten Fällen erhält man bei der Reaktion einen kristallinen Niederschlag, welcher direkt abgesaugt wird und der sich aus einem geeigneten Lösungsmittel, wie z. B. Ethanol/Wasser umkristallisieren läßt. Die Mutterlauge engt man anschließend ein und arbeitet sie destillativ zur Gewinnung der restlichen Mengen I auf.

Bei Verwendung von Alkanolen als Lösungsmittel können als Hauptprodukte die Resorcinderivate Ia, deren Monoether Ib und/oder die Bisether Ic entstehen.

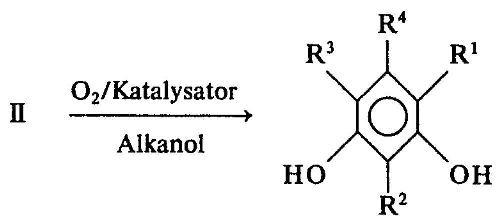


(Ia)

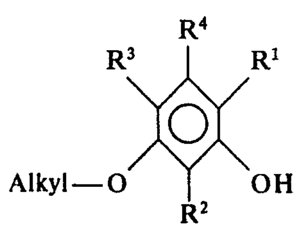

(Ib)

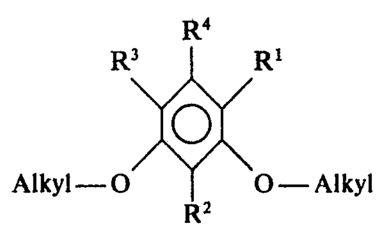

(Ic)

Ausbeute und Art bzw. die Verteilung der bei dem erfindungsgemäßen Verfahren entstehenden Produkte Ia–c hängen in erster Linie von der Art der Substituenten $R^1$ bis $R^4$ in Formel I sowie von der Art des als Lösungsmittel verwendeten Alkanols ab.

Besonders vorteilhaft lassen sich nach dem erfindungsgemäßen Verfahren 3,6-Dialkyl-$\beta$-dihydroresorcylsäureester oder die entsprechenden Nitrile (allgemeine Formel II, worin $R^1$ = Alkoxycarbonyl oder Nitril, $R^2$ und $R^4$ = Alkyl und $R^3$ = Wasserstoff ist) aromatisieren. Man verwendet hierzu bevorzugt Kupferverbindungen in Verbindung mit Chlorwasserstoff oder Thionylchlorid als Katalysatorsystem und Methanol als Lösungsmittel. Als Aromatisierungsprodukt erhält man dann aus 3,6-Dimethyl-$\beta$-dihydroresorcylsäuremethylester den Rhizoninsäuremethylester VI vom Typ Ib mit maximalen Ausbeuten von über 80% d.Th. neben ca. 10% 3,6-Dimethyl-$\beta$-resorcylsäuremethylester VII vom Typ Ia

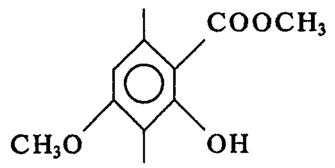

VI

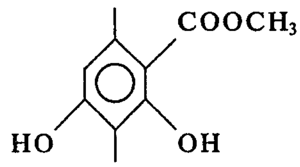

VII

Dagegen läßt sich beispielsweise Cyclohexan-1,3-dion ($R^2$ bis $R^4$ = H) an einem $Cu_2O$/Thionylchlorid-Katalysator in methanolischer Lösung in 76%iger Ausbeute in das 1,3-Dimethoxybenzol vom Typ Ic überführen. Unter gleichen Bedingungen erhält man aus 5-Methyl-cyclohexan-1,3-dion in 60%iger Ausbeute 1,3-Dimethoxy-5-methyl-benzol als Hauptprodukt.

Verwendet man bei der Umsetzung von 3,6-Dialkyl-$\beta$-dihydroresorcylsäureestern anstelle von Methanol Isopropanol oder tert.-Butanol als Lösungsmittel, so bilden sich in stärkerem Maße die 3,6-Dimethyl-$\beta$-resorcylsäureester mit unveretherten Hydroxylgruppen (VII), die im Extremfall sogar zum Hauptprodukt werden können.

In Gegenwart von niedermolekularen primären und sekundären Alkoholen treten neben der Verbindung VII Produkte vom Typ der allgemeinen Formel Ib auf, in denen der Ether- und Esterrest gleich oder aber verschieden sind. In Gegenwart von Methyl-tert.-butylether bilden sich vorzugsweise Verbindungen vom Typ Ib, in denen Alkyl für Methyl steht.

Beabsichtigt man, ausschließlich Resorcinderivate mit unveretherten Hydroxylgruppen herzustellen, so empfiehlt es sich in Tetrahydrofuran als Lösungsmittel zu arbeiten.

Der 3,6-Dimethyl-$\beta$-resorcylsäuremethylester VII stellt als Inhaltsstoff des natürlichen Eichenmooses ein wichtiges Handelsprodukt für die Parfümerie dar. Es wurde gefunden, daß der Monomethylether VI diesem Produkt hinsichtlich Geruchsqualität und Geruchsstärke vergleichbar ist und auch das entsprechende Nitril ein guter Riechstoff mit Eichenmooscharakter ist.

So bietet das erfindungsgemäße Verfahren die Möglichkeit neben begehrten Geruchsträgern des natürlichen Eichenmooses auch Resorcinderivate mit vergleichbar guten Riechstoffeigenschaften und darüber hinaus Resorcinderivate der allgemeinen Formeln Ia–Ic auf einfache Weise und in guten Ausbeuten herzustellen.

## Beispiel 1

Zu einer Lösung von 9,9 g (50 mmol) 3,6-Dimethyl-$\beta$-dihydroresorcinsäuremethylester in 100 ml Methanol gibt man 11,9 g (0,1 mol) Thionylchlorid in 1 g (7 mmol) Kupfer-(I)-oxid. In dieses Reaktionsgemisch leitet man 6 Stunden lang unter Rühren bei 50°C einen Luftstrom von 45 Liter/Stunde ein. Danach engt man das Reaktionsgemisch auf ein Drittel seines Volumens ein und läßt über Nacht bei 0 bis 5°C stehen. Anschließend sammelt man den kristallinen Niederschlag auf einem Büchnertrichter. Nach dem Waschen der Kristalle mit kaltem Methanol und Trocknen erhält man 7,63 g 3,6-Dimethyl-2-hydroxy-4-methoxy-benzoesäuremethylester VI (Rhizoninsäuremethylester) vom Schmelzpunkt F = 87 bis 90°C.

Die Mutterlauge wird vom Lösungsmittel befreit und der Rückstand destilliert. Bei 100 bis 120°C/0,1 mbar gehen 2,61 g eines Produktgemisches über, welches laut gaschromatographischer Analyse (2 m OV-17; 200°C) zu 20,4% aus 3,6-Dimethyl-$\beta$-resorcylsäuremethylester VII und zu 46,8% aus Rhizoninsäuremethylester VI besteht. Gesamtausbeute an Rhizoninsäuremethylester VI: 8,85 g (42 mmol) = 84% d.Th. und 3,6-Dimethyl-$\beta$-resorcylsäuremethylester VII: 0,53 g (3 mmol) = 6% d.Th. Rhizoninsäuremethylester: 1 H-NMR-Spektrum ($CDCl_3$) $\delta$ = 2,08 (s, $-CH_3$), 2,50 (s, $-CH_3$), 3,85 (s, $-CH_3$), 3,90 (s, $-CH_3$), 6,25 (s. H), 11,75 ppm (OH)

Duftnote von Rhizoninsäuremethylester: Eichenmoos; vergleichbar der von VII.

## Beispiel 2

Bei Raumtemperatur leitet man 250 g Chlorwasserstoffgas in 3 l Methanol ein, löst 198 g (1 mol) 3,6-Dimethyl-dihydro-β-resorcylsäuremethylester in dem erhaltenen HCl-Methanol-Gemisch auf und fügt 20 g (0,14 mol) $Cu_2O$ zu. In dieses Reaktionsgemisch leitet man bei 50°C 11 Stunden lang unter Rühren einen Luftstrom von 75 l/h ein. Arbeitet man das Reaktionsprodukt analog Beispiel 1 auf, so erhält man insgesamt 166,6 g (0,79 mol) entsprechend 79% d. Th. an Rhizoninsäuremethylester VI, wovon 150 g in Form reiner Kristalle anfallen, und 26,1 g (0,13 mol) entsprechend 13% d. Th. an 3,6-Dimethyl-β-resorcyl-säuremethylester VII.

## Beispiel 3 bis 10

Oxidation von 3,6-Dimethyl-dihydro-β-resorcylsäuremethylester (IIa) unter variablen Bedingungen analog Beispiel 1 und 2. Nähere Angaben über eingesetzte Ausgangsverbindungen, Reaktionsbedingungen und Ausbeuten an Rhizoninsäuremethylester (VI) sowie an 3,6-Dimethyl-β-resorcylsäuremethylester (VII) finden sich in der folgenden Tabelle 1. Beispiel 4 wird mit reinem Sauerstoff durchgeführt.

Tabelle 1

| | Beispiel 3 | 4 | 5 | 6 | 7 | 8 | 9 | 10 |
|---|---|---|---|---|---|---|---|---|
| Ausgangsverbindung (IIa) [mol] | 0,05 | 0,05 | 0,05 | 0,05 | 0,05 | 0,05 | 0,05 | 0,05 |
| Katalysator | $Cu_2O$ | $Cu_2O$ | CuCl | $CuCl_2 \times 2\ H_2O$ | $CuBr_2$ | $CuAc_2$ | $Cu_2O$ | $Cu_2O$ |
| Menge [mmol] | 7 | 7 | 10 | 6 | 4,5 | 5,5 | 7 | 7 |
| Hilfsreagens | HCl | HCl | HCl | HCl | HCl | HCl | HBr | $SOCl_2$ |
| Menge [g] | 12 | 13 | 12 | 12 | 16 | 11 | 8 | 12 |
| Lösungsmittel | MeOH | MeOH | MeOH | MeOH | MeOH | MeOH | MeOH | 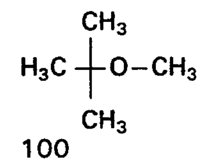 |
| Menge [ml] | 100 | 100 | 100 | 100 | 100 | 100 | 100 | 100 |
| Luftstrom (bzw. $O_2$ bei$^+$) [l/h] | 10 | $10^+$ | 45 | 45 | 45 | 45 | 45 | 45 |
| Reaktionstemp. [°C] | 50 | 50 | 50 | 50 | 50 | 50 | 50 | 50 |
| Reaktionsdauer [h] | 12 | 5 | 7 | 5 | 4 | 11 | 13 | 4,5 |
| Ausbeute an VI [% d.Th.] | 81,2 | 67,6 | 77,5 | 52,8 | 66,6 | 51,0 | 64,0 | 76,9 |
| Ausbeute an VIII [% d.Th.] | 10,0 | 8,1 | 9,3 | 8,4 | 15,2 | 8,5 | 2,7 | 12,6 |

## Beispiel 11

9,9 g (0,05 mol) 3,6-Dimethyl-dihydro-$\beta$-resorcylsäuremethylester werden analog Beispiel 2 in Gegenwart von 1 g (6 mmol) $CuCl_2 \cdot 2\ H_2O$ und 12 g Chlorwasserstoff in Lösung von 100 ml n-Amylalkohol umgesetzt. Reaktionstemperatur 50°C; Luftstrom 45 l/h; Reaktionszeit 6 h; Ausbeute an VII: 18% d.Th. Ausbeute an 3,6-Dimethyl-2-hydroxy-4-amyloxy-benzoesäuremethylester vom Schmp. 67 bis 68°C: 72,5% d.Th.; 1 H-NMR-Spektrum ($CDCl_3$): $\delta$ = 1,05 (+, $-CH_3$), 1,1-2 (m-$CH_2$), 2,04 (s, $-CH_3$), 2,46 (s, $-CH_3$), 3,82 (s, $-CH_3$), 3,9 (+, $-CH_2$), 6,12 (s, H), 11,47 ppm (s, OH).

## Beispiel 12

9,9 g (0,05 mol) 3,6-Dimethyl-dihydro-$\beta$-resorcylsäuremethylester werden analog Beispiel 2 in Gegenwart von 1 g (7 mmol) $Cu_2O$ und 15 g Chlorwasserstoff in Lösung von 100 ml Isopropanol umgesetzt, Reaktionstemperatur 50°C, Luftstrom 45 l/h, Reaktionszeit 8 h. Ausbeute an VII: 38,1% d.Th.; Ausbeute an 3,6-Dimethyl-2-hydroxy-4-isopropoxy-benzosäuremethylester vom Schmp. 78 bis 80°C: 48,5% d.Th.; 1 H-NMR-Spektrum ($CDCl_3$): $\delta$ = 1,30 (d, 2-$CH_2$), 2,01 (s, $-CH_3$), 2,42 (s, $-CH_3$), 3,80 (s, $-CH_3$), 4,48 (m), 6,12 (s, $-H$), 11,66 ppm (s, OH).

## Beispiel 13

8,3 g (0,05 mol) 3,6-Dimethyl-2,4-dioxo-cyclohexannitril werden in Gegenwart von 1 g (7 mmol) $Cu_2O$ und 15 g Chlorwasserstoffgas in Lösung von 100 ml Methanol umgesetzt. Reaktionstemperatur 50°C; Luftstrom 45 l/h; Reaktionszeit 9 h. Ausbeute an 3,6-Dimethyl-2,4-dihydroxy-benzonitril: 14,7% d.Th.; Ausbeute an 3,6-Dimethyl-2-hydroxy-4-methoxy-benzonitril vom Schmp. 155 bis 157°C: 58,6% d.Th.; 1 H-NMR-Spektrum ($CDCl_3$): $\delta$ = 2,0 (s, $-CH_3$), 2,36 (s, $-CH_3$), 3,81 (s, $-CH_3$), 6,56 (s, $-H$), 9,72 ppm (s, $-OH$).
Duftnote:

## Beispiel 14

17 g (0,1 mol) Dihydro-$\beta$-resorcylsäuremethylester werden in Gegenwart von 2 g (14 mmol) $Cu_2O$ und 24 g (0,2 mol) Thionylchlorid in Lösung von 200 ml Methanol analog Beispiel 1 umgesetzt. Reaktionstemperatur 50°C; Luftstrom 45 l/h; Reaktionszeit 13 h. Ausbeute an 1,3-Dimethoxy-benzol: 7% d.Th.; Ausbeute an 2-Hydroxy-4-methoxy-benzoesäuremethylester vom Schmp. 47 bis 48°C: 21% d.Th.; 1 H-NMR-Spektrum ($CDCl_3$): 2,51 (s, $-CH_3$), 3,90 (s, $-CH_3$), 3,95 (s, $-CH_3$), 6,30 (s, 2 H), 12,2 ppm (s, OH): Ausbeute an 2,4-Dimethoxy-benzoesäuremethylester 27,9% d.Th.; 1 H-NMR-Spektrum ($CDCl_3$): $\delta$ = 3,80 (s, $-CH_3$), 3,82 (s, $-CH_3$), 3,86 (s, $-CH_3$), 6,45 (m, 2 H), 7,78 ppm (m, H); $n_D^{25}$ = 1,5432.

## Beispiel 15

11,2 g (0,1 mol) Cyclohexan-1,3-dion werden in Gegenwart von 2 g (14 mmol) $Cu_2O$ und 24 g (0,2 mol) Thionylchlorid in Lösung von 200 ml Methanol analog Beispiel 1 umgesetzt. Reaktionstemperatur 50°C, Luftstrom 45 l/h; Reaktionszeit 10 h. Ausbeute an 1,3-Dimethoxy-benzol: 76% d.Th.; 1 H-NMR-Spektrum ($CDCl_3$) = 3,78 (s, 2-$CH_3$), 6,49 (m, 3 H), 7,18 ppm (m, 1 H).

## Beispiel 16

6,3 g (0,05 mol) 5-Methyl-cyclohexan-1,3-dion werden in Gegenwart von 1 g (7 mmol) $Cu_2O$ und 12 g (0,1 mol) Thionylchlorid in Lösung von 100 ml Methanol analog Beispiel 1 umgesetzt. Reaktionstemperatur 50°C; Luftstrom 45 l/h; Reaktionszeit 8 h. Ausbeute an 1,3-Dimethoxy-5-methyl-benzol: 60% d.Th.; Massenspektrum (70eV): n/e = 152 (100%, $M^+$), 137 (4%), 123 (44%), 109 (13%), 31 (22%), 77 (18%), 66 (10%).

## Beispiel 17

In 200 ml Tetrahydrofuran (TMF) werden bei 25°C 25 g Chlorwasserstoffgas eingeleitet. Zu dieser Lösung werden 2 g $Cu_2O$ und 19,8 g 3,6-Dimethyl-$\beta$-dihydro-resorcinsäuremethylester addiert. Anschließend wird das Reaktionsgemisch auf 50°C erhitzt und ein Luftstrom von 45 Litern/Stunde eingeleitet. Nach 7 Stunden ist alles Ausgangsmaterial umgesetzt.

Das Reaktionsgemisch wird eingeengt und auf ein Wasser/Toluol-Gemisch gegossen, die abgetrennte organische Phase mit Wasser neutral gewaschen, eingeengt und schließlich über eine Brücke destilliert. Man erhält den 3,6-Dimethyl-$\beta$-resorcylsäuremethylester VII in einer Ausbeute von 53,8% der Theorie.

Beispiel 18

a) Unter erfindungsgemäßen Bedingungen

Bei Raumtemperatur leitet man 12 g Chlorwasserstoffgas in 100 ml Methanol ein, löst 9,9 g (0,05 mol) 3,6-Dimethyldihydro-$\beta$-resorcylsäuremethylester in dem erhaltenen HCl-Methanol-Gemisch auf und fügt 1 g (7 mmol) $Cu_2O$ zu.
In dieses Reaktionsgemisch leitet man bei 50°C einen Luftstrom von 45 Litern/Stunde ein bis laut dünnschichtchromatographischer Analyse alle Ausgangsverbindung umgesetzt ist.
Das Reaktionsgemisch wird auf 1/3 des Volumens eingeengt, auf 5°C abgekühlt und die sich ausscheidenden Kristalle abgetrennt. Die Mutterlauge wird auf ein Wasser/Toluol-Gemisch gegeben, die abgetrennte organische Phase mit Wasser neutral gewaschen, eingeengt und schließlich über eine Brücke destilliert.
Man erhält Rhizoninsäuremethylester (VI) in einer Gesamtausbeute von 81 % der Theorie und 3,6-Dimethyl-$\beta$-resorcylsäuremethylester (VII) in einer Gesamtausbeute von 10% der Theorie.

b) Vergleichsbeispiel unter Zusatz von 5 ml Wasser zum Reaktionsgemisch

Arbeitet man wie unter a) beschrieben, verwendet jedoch anstelle von 100 ml Methanol ein Gemisch aus 100 ml Methanol und 5 ml Wasser, so erhält man Rhizoninsäuremethylester (VI) in einer Gesamtausbeute von 34,9% der Theorie und 3,6-Dimethyl-$\beta$-resorcylsäuremethylester (VII) in einer Gesamtausbeute von 18%.

c) Vergleichsbeispiel unter Zusatz von 10 ml Wasser zum Reaktionsgemisch

Arbeitet man wie unter a) beschrieben, verwendet jedoch als Lösungsmittel anstelle von 100 ml Methanol ein Gemisch aus 100 ml Methanol und 10 ml Wasser, so erhält man Rhizoninsäuremethylester in einer Gesamtausbeute von nur 34,3% der Theorie und 3,6-Dimethylsäuremethylester (VII) in einer Ausbeute von 15,9% der Theorie.

**Patentansprüche**

1. Verfahren zur Herstellung von Resorcinderivaten der allgemeinen Formel I

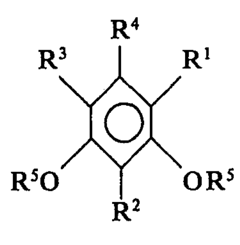

(I)

in welcher $R^1$ eine Alkoxycarbonylgruppe mit einem niederen Alkylrest, eine Nitrilgruppe, einen niederen Alkylrest oder Wasserstoff bedeutet und $R^2$ bis $R^5$ für Wasserstoff oder einen niederen Alkylrest stehen, dadurch gekennzeichnet, daß man die entsprechenden Cyclohexan-1,3-dione der allgemeinen Formel II

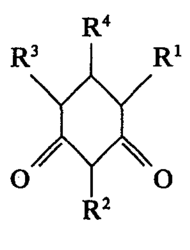

(II)

in welcher die Substituenten die oben angegebene Bedeutung haben, in Gegenwart katalytischer Mengen einer Kupferverbindung sowie in Gegenwart von 1 bis 10 mol eines Halogenwasserstoffgases oder von 0,5 bis 5 mol Thionylchlorid pro mol II in einem praktisch wasserfreien Alkanol mit 1 bis 6 C-Atomen, praktisch wasserfreiem Tetrahydrofuran oder praktisch wasserfreiem Methyl-tert.-butylether als Lösungsmittel bei Reaktionstemperaturen von 0 bis 150°C mit Sauerstoff oder Sauerstoff enthaltenden Gasen umsetzt.

2. Verfahren zur Herstellung von Resorcinderivaten der allgemeinen Formel I gemäß Anspruch 1, dadurch gekennzeichnet, daß man die Umsetzung in Gegenwart katalytischer Mengen von Kupfer(I)oxid durchführt.

3. Verfahren zur Herstellung von Resorcinderivaten der allgemeinen Formel I gemäß Anspruch 1, dadurch gekennzeichnet, daß man die Umsetzung in Gegenwart von Chlorwasserstoffgas, Bromwasserstoffgas oder von Thionylchlorid durchführt.

4. Verfahren zur Herstellung von Resorcinderivaten der allgemeinen Formel I gemäß Anspruch 1, dadurch gekennzeichnet, daß man die Umsetzung bei Reaktionstemperaturen von 20 bis 100°C durchführt.

5. Verwendung von Resorcinderivaten der allgemeinen Formel III

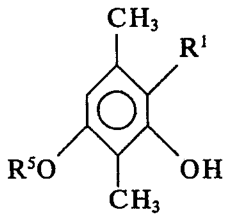

(III)

in welcher $R^1$ eine Alkoxycarbonylgruppe mit einem niederen Alkylrest oder eine Nitrilgruppe und $R^5$ einen niederen Alkylrest bedeuten, als Riechstoffe.

**Claims**

1. A process for the preparation of a resorcinol derivative of the general formula I

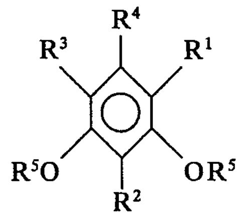

(I)

where $R^1$ is lower alkoxycarbonyl, nitrile, lower alkyl or hydrogen, and $R^2$ to $R^5$ are hydrogen or lower alkyl, wherein the corresponding cyclohexane-1,3-dione of the general formula II

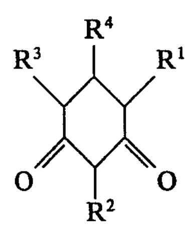

(II)

where the substituents have the above meanings, is reacted with oxygen or an oxygen-containing gas in the presence of a catalytic amount of a copper compound and in the presence of from 1 to 10 moles of a gaseous hydrogen halide, or of from 0,5 to 5 moles of thionyl chloride, per mole of II, in a substantially anhydrous alkanol of from 1 to 6 carbon atoms, substantially anhydrous tetrahydrofuran or substantially anhydrous methyl tert.-butyl ether as the solvent, at from 0 to 150°C.

2. A process for the preparation of a resorcinol derivative of the general formula I as claimed in claim 1, wherein the reaction is carried out in the presence of a catalytic amount of copper(I) oxide.

3. A process for the preparation of a resorcinol derivative of the general formula I as claimed in claim 1, wherein the reaction is carried out in the presence of hydrogen chloride, hydrogen bromide or thionyl chloride.

4. A process for the preparation of a resorcinol derivative of the general formula I as claimed in claim 1, wherein the reaction is carried out at from 20 to 100°C.

5. The use of a resorcinol derivative of the general formula III

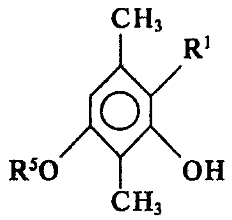

(III)

where $R^1$ is lower alkoxycarbonyl or nitrile and $R^5$ is lower alkyl, as a scent.

**Revendications**

1. Procédé de préparation de dérivés du résorcinol de formule générale I

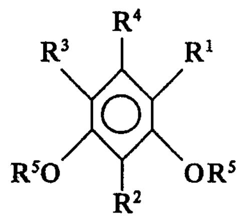

(I)

dans laquelle $R^1$ représente un groupe alcoxycarbonyle contenant un groupe alkyle inférieur, un groupe nitrile, un groupe alkyle inférieur ou l'hydrogène et $R^2$ à $R^5$ représentent l'hydrogène ou un groupe alkyle inférieur, caractérisé en ce que l'on fait réagir les cyclohexane-1,3-diones correspondantes de formule générale II

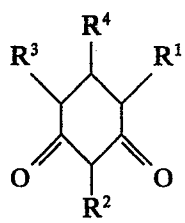

(II)

dans laquelle les substituants ont les significations indiquées ci-dessus, en présence de quantités catalytiques d'un composé du cuivre et en présence de 1 à 10 moles d'un halogénure d'hydrogène gazeux ou de 0,5 à 5 moles de chlorure de thionyle par mole du composé II dans un alcanol en C 1—C 6 pratiquement anhydre, du tétrahydrofuranne pratiquement anhydre ou l'oxyde de méthyle et de tert.-butyle pratiquement anhydre servant de solvant, à des températures de réaction de 0 à 150°C, avec l'oxygène ou des gaz contenant de l'oxygène.

2. Procédé de préparation des dérivés du résorcinol de formule générale I selon la revendication 1, caractérisé en ce que l'on effectue la réaction en présence de quantités catalytiques d'oxyde cuivreux.

3. Procédé de préparation de dérivés du résorcinol de formule générale I selon la revendication 1, caractérisé en ce que l'on effectue la réaction en présence de chlorure d'hydrogène gazeux, de bromure d'hydrogène gazeux ou de chlorure de thionyle.

4. Procédé de préparation de dérivés du résorcinol de formule générale I selon la revendication 1, caractérisé en ce que l'on effectue la réaction à des températures de 20 à 100°C.

5. Utilisation de dérivés du résorcinol de formule générale III

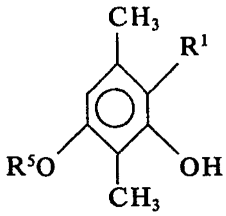

(III)

dans laquelle $R^1$ représente un groupe alcoxycarbonyle contenant un groupe alkyle inférieur ou un groupe nitrile et $R^5$ représente un groupe alkyle inférieur, en tant que parfums.